# EUROPEAN PATENT APPLICATION

(11) **EP 4 286 509 A1**
(43) Date of publication of application: **06.12.2023**
(21) Application number: 23754691.6
(22) Date of filing: 06.03.2023
(51) Int. Cl.: C12M 1/34, C12M 1/00, C12Q 1/6844

(54) **NUCLEIC ACID DETECTION APPARATUS AND NUCLEIC ACID DETECTION METHOD**

(30) Priority: 18.04.2022 CN 202210400960
(71) Applicant: Assure Tech. (Hangzhou) Co., Ltd., Hangzhou, Zhejiang 310000 (CN)
(72) Inventor: LING, Shisheng, Hangzhou, Zhejiang 310000 (CN); LI, Jun, Hangzhou, Zhejiang 310000 (CN); WANG, Liang, Hangzhou, Zhejiang 310000 (CN); CHEN, Dong, Hangzhou, Zhejiang 310000 (CN)
(74) Representative: Wang, Bo
(86) International application number: PCT/CN2023/079823
(87) International publication number: WO 2023/202246

(57) **Abstract**

The present disclosure provides nucleic acid detection devices and nucleic acid detection methods. The nucleic acid detection devices may include a housing portion and a reaction portion. The housing portion may be configured to include a sample chamber, a first observation chamber and a second observation chamber, and the reaction portion may be configured to include a first reaction chamber and a second reaction chamber. The sample chamber may communicate with the first reaction chamber, and the sample chamber may communicate with the second reaction chamber. The first observation chamber may communicate with the first reaction chamber, and the second observation chamber may communicate with the second reaction chamber.

## Description

### CROSS-REFERENCE TO RELATED APPLICATIONS

This application claims priority to Chinese Patent Application No. 202210400960.1, filed on April 18, 2022, the contents of which are incorporated herein by reference.

### TECHNICAL FIELD

The present disclosure generally relates to the field of medical diagnosis, in particular to nucleic acid detection devices and nucleic acid detection methods.

### BACKGROUND

Nucleic acid detection is a form medical detection methods for in-vitro diagnosis, and it enables the rapid detection of nucleic acids of pathogens in test samples. The nucleic acid detection is the most direct, reliable, and sensitive approach for rapid and specific detection of pathogens in an early stage, and has important applications in the fields of disease diagnosis, epidemic prevention and control, health monitoring, etc. By detecting a genetic material (e.g., DNA or RNA) of pathogens, the nucleic acid detection can provide information (e.g., a type, a concentration, etc.) about the pathogens, thereby avoiding interference from factors such as the disease infection window period, etc. Current nucleic acid detections usually utilize devices that are based on real-time fluorescence quantitative polymerase chain reaction (PCR) technology. However, the nucleic acid detection devices are often complex to control, take too long to be operational, and have strict requirements for operators and environments, thereby greatly limiting the groups of operators that are eligible to use the nucleic acid detection devices and the environments in which the nucleic acid detection devices can be used.

Therefore, the technical problems that urgently need to be solved in the field would include how to make the nucleic acid detection devices capable of easily and quickly detecting the nucleic acids of pathogens and how to improve the detection efficiency in the medical diagnosis process.

### SUMMARY

Some embodiments of the present disclosure provide a nucleic acid detection device. The nucleic acid detection device may include a housing portion and a reaction portion. The housing portion may be configured to include a sample chamber, a first observation chamber, and a second observation chamber, and the reaction portion may be configured to include a first reaction chamber and a second reaction chamber. The sample chamber may communicate with the first reaction chamber, and the sample chamber may communicate with the second reaction chamber. The first observation chamber may communicate with the first reaction chamber, and the second observation chamber may communicate with the second reaction chamber.

Some embodiments of the present disclosure further provide a nucleic acid detection method, utilizing to the nucleic acid detection device described in any one of the technical solutions of the present disclosure. The nucleic acid detection method may include placing a sample into the sample chamber; making fluid containing the sample to enter the first reaction chamber through the first sample outlet, and making the fluid containing the sample to enter the second reaction chamber through the second sample outlet; making the sample to undergo a reverse transcription loop-mediated isothermal amplification reaction within the first reaction chamber to detect an internal standard gene of the sample, and making the sample to undergo a reverse transcription loop-mediated isothermal amplification reaction within the second reaction chamber to detect a target gene of the sample; making a first amplification reaction product generated after the reverse transcription loop-mediated isothermal amplification reaction occurs in the first reaction chamber to enter the first observation chamber, and making a second amplification reaction product generated after the reverse transcription loop-mediated isothermal amplification reaction occurs in the second reaction chamber to enter the second observation chamber; and determining a color of the first amplification reaction product in the first observation chamber, and determining a color of the second amplification reaction product in the second observation chamber.

### BRIEF DESCRIPTION OF THE DRAWINGS

The present disclosure is further described in terms of exemplary embodiments. These exemplary embodiments are described in detail with reference to the drawings. These embodiments are non-limiting exemplary embodiments, in which like reference numerals represent similar structures throughout the several views of the drawings, and wherein:
FIG. 1 is a schematic diagram illustrating an exemplary structure of a nucleic acid detection device according to some embodiments of the present disclosure;
FIG. 2 is a schematic diagram illustrating an exemplary assembly of a nucleic acid detection device according to some embodiments of the present disclosure;
FIG. 3 is a sectional view illustrating an exemplary nucleic acid detection device according to some embodiments of the present disclosure;
FIG. 4 is a bottom view illustrating a housing portion according to some embodiments of the present disclosure;
FIG. 5 is a bottom view illustrating a reaction portion according to some embodiments of the present disclosure;
FIG. 6 is a schematic diagram illustrating an exemplary structure of a piston assembly according to some embodiments of the present disclosure; and
FIG. 7 is a flowchart illustrating a nucleic acid detection process according to some embodiments of the present disclosure.

In the drawings, 100 represents a nucleic acid detection device, 110 represents a housing portion, 120 represents a reaction portion, 111 represents a sample chamber, 111-1 represents a first sample outlet, 111-2 represents a second sample outlet, 111-3 represents a sample inlet, 112 represents a piston chamber, 1121 represents a fifth sub-chamber, 1122 represents a sixth sub-chamber, 112-1 represents a first opening, 112-2 represents a second opening, 113 represents a first observation chamber, 114 represents a second observation chamber, 113-1 represents a first inlet, 113-2 represents a first observation port, 114-1 represents a second inlet, 114 -2 represents a second observation port, 121 represents a first reaction chamber, 122 represents a second reaction chamber, 1211 represents a first sub-chamber, 1212 represents a second sub-chamber, 1212-1 represents a first portion, 1212-2 represents a second portion, 1221 represents a third sub-chamber, 1222 represents a fourth sub-chamber, 1222-1 represents a third portion, 1222-2 represents a fourth portion, 130 represents a piston assembly, 131 represents a first piston, 132 represents a second piston, 133 represents a connector, 134 represents a first piston rod, 135 represents a second piston rod, 141 represents a first way valve structure, 142 represents a second way valve structure, 143 represents a third way valve structure, 144 represents a fourth way valve structure, 150 represents a hydrophobic breathable membrane, 160 represents an elastic membrane, 161 represents a first through hole, 162 represents a second through hole, 163 represents a third through hole, 164 represents a fourth through hole, 165 represents a fifth through hole, 166 represents a sixth through hole, and 170 represents a sealing membrane.

### DETAILED DESCRIPTION

In order to illustrate the technical solutions related to the embodiments of the present disclosure, a brief introduction of the drawings referred to the description of the embodiments is provided below. Obviously, drawings described below are only some examples or embodiments of the present disclosure. Those having ordinary skills in the art, without further creative efforts, may apply the present disclosure to other similar scenarios according to these drawings. Unless apparent from the locale or otherwise stated, like reference numerals represent similar structures or operations in the drawings.

The terms "first," "second," and similar words used in this description and the claims do not indicate any order, number, or importance, but are only used to distinguish different components. Similarly, the terms "an," "one," and similar words do not indicate a numerical limitation, but indicate the presence of at least one. Unless otherwise indicated, the terms "top," "bottom," and similar words are merely for illustrative purposes and are not restricted to a location or a spatial orientation. In general, the terms "comprise" and "include" merely prompt to include steps and elements that have been clearly identified, and these steps and elements do not constitute an exclusive listing. The methods or devices may also include other steps or elements.

Some embodiments of the present disclosure relate to a nucleic acid detection device and a nucleic acid detection method. A sample chamber of the nucleic acid detection device may be configured to receive and accommodate a sample. A first reaction chamber and a second reaction chamber may be configured to receive the sample within the sample chamber and make the sample to react (e.g., perform an amplification reaction), thereby achieving a nucleic acid detection of the sample (e.g., a detection of an internal standard gene and/or a detection of a target gene, etc.). A first observation chamber and a second observation chamber may be configured to observe fluid (e.g., an amplification reaction product) after the reaction within the first reaction chamber and the second reaction chamber. In addition, the first reaction chamber and the second reaction chamber may be configured to respectively perform the different types of nucleic acid detections on the sample at the same time. For example, the first reaction chamber may be configured to detect the internal standard gene and the second reaction chamber may be configured to detect the target gene. By using the nucleic acid detection device of the present disclosure for the nucleic acid detection, the first reaction chamber and the second reaction chamber may be configured to make the sample to undergo different reactions at the same time so that different detections can be performed at the same time, while the first observation chamber and the second observation chamber may be configured to visually observe the fluid after the reactions occur in the first reaction chamber and the second reaction chamber, respectively, to obtain results of the nucleic acid detections, thereby improving the detection efficiency. And the nucleic acid detection device may be small in size, light in weight, and easy to operate, so that the nucleic acid detection device can easily and quickly achieve the nucleic acid detection of the sample. The nucleic acid detection device and the nucleic acid detection method of the present disclosure may be applied to perform the nucleic acid detection on viruses (e.g., novel coronavirus) and bacteria, etc.

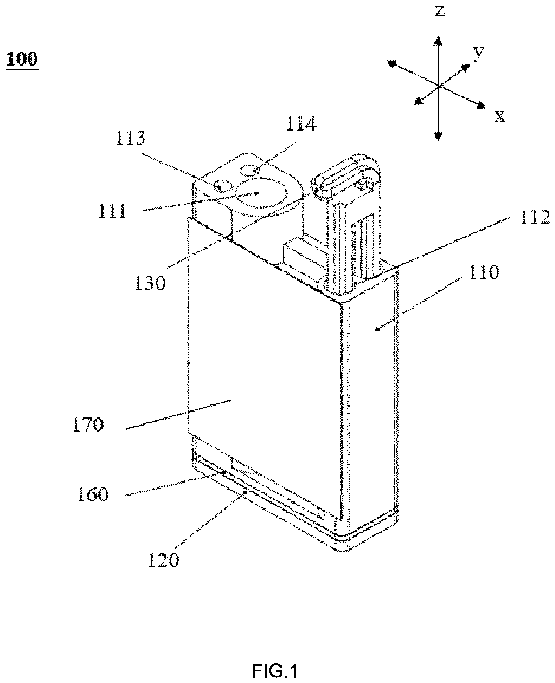

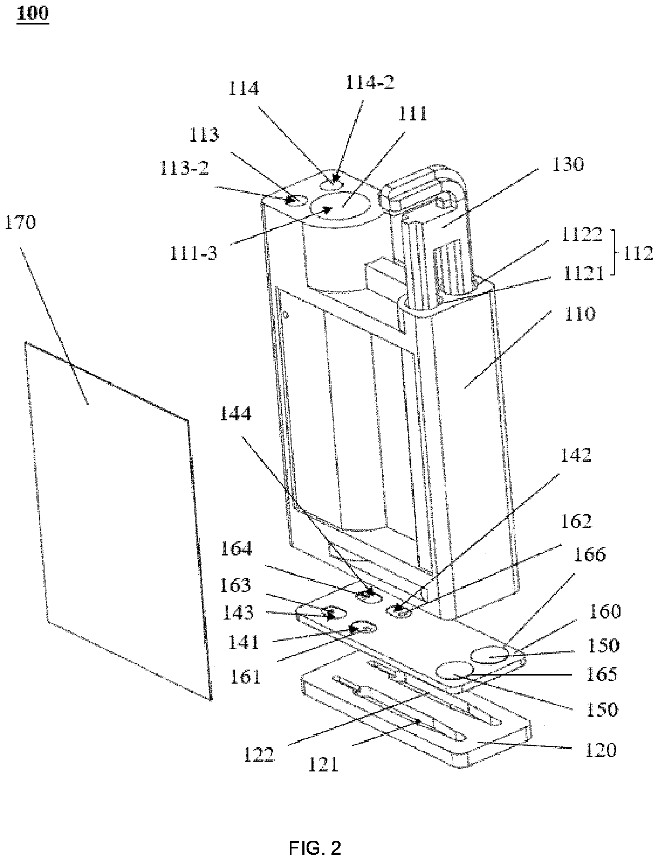

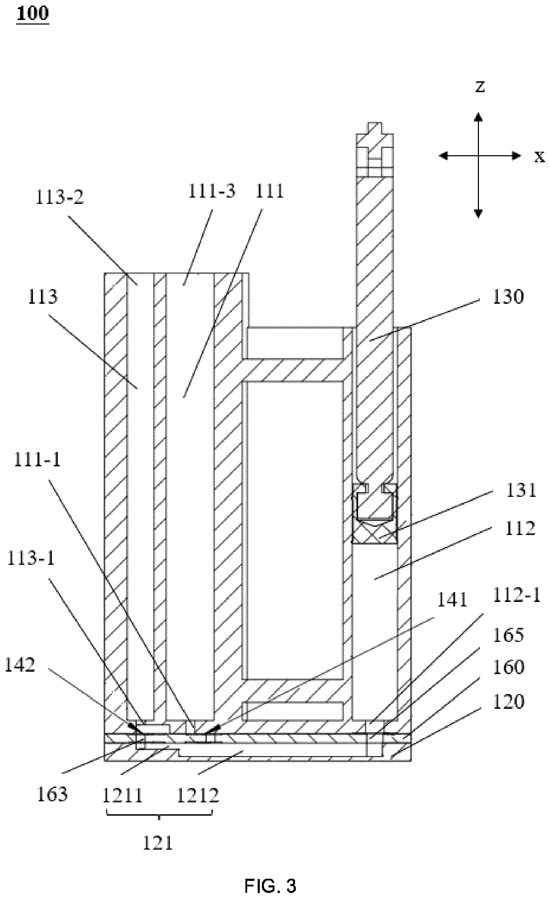

FIG. 1 is a schematic diagram illustrating an exemplary structure of a nucleic acid detection device according to some embodiments of the present disclosure. FIG. 2 is a schematic diagram illustrating an exemplary assembly of a nucleic acid detection device according to some embodiments of the present disclosure. FIG. 3 is a sectional view illustrating an exemplary nucleic acid detection device according to some embodiments of the present disclosure. Referring to FIGs. 1-3, a nucleic acid detection device 100 may include a housing portion 110 and a reaction portion 120. The housing portion 110 may be configured to include a sample chamber 111, a first observation chamber 113, and a second observation chamber 114. The reaction portion 120 may be configured to include a first reaction chamber 121 and a second reaction chamber 122. The sample chamber 111 may communicate with the first reaction chamber 121, and the sample chamber 111 may communicate with the second reaction chamber 122. The first observation chamber 113 may communicate with the first reaction chamber 121, and the second observation chamber 114 may communicate with the second reaction chamber 122. In some embodiments, as illustrated in FIG. 1, the reaction portion 120 may be located at a bottom portion of the housing portion 110. In some other embodiments, the reaction portion 120 may be located in other orientations of the housing portion 110, such as, a top portion, a side portion, etc.

The sample chamber111 may be a columnar structure with a hollow interior. A top portion of the sample chamber 111 may be configured to include an opening (e.g., a sample inlet 111-3 as described below) for receiving a sample, and a bottom portion of the sample chamber 111 may also be configured to include one or more openings (e.g., a first sample outlet 111-1 and a second sample outlet 111-2 as described below) for communicating with the first reaction chamber 121 and the second reaction chamber 122. In some embodiments, the sample chamber 111 may be used to accommodate the sample, such as, a swab-type sample, etc. The sample may be lysed and released in the sample chamber 111, so that a nucleic acid detection template may be obtained. In some embodiments, a sample release agent may be also accommodated inside the sample chamber 111. The sample release agent may be used to make the sample in the sample chamber 111 to undergo the lysis and release to obtain the nucleic acid detection template. In some embodiments, when the nucleic acid detection device 100 is used for the nucleic acid detection, the sample release agent may be placed in the sample chamber 111 in advance and the sample may be lysed and released under the action of the sample release agent, so that the nucleic acid detection template of the sample may be obtained. In some embodiments, a formula of the sample release agent may include a non-ionic surfactant of 0.2-2%, trehalose of 0.01-2%, potassium chloride of 50-200 mmol/L, ethylene diamine tetraacetic acid of 1-10 mmol/L, Tri-HCI of 0.2-5 mmol/L, or the like, or any combination thereof.

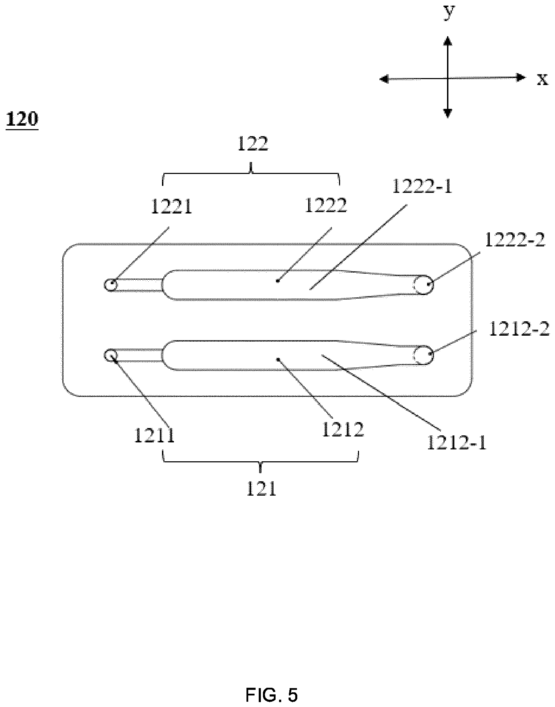

In some embodiments, the sample may undergo an amplification reaction (e.g., a reverse transcription loop-mediated isothermal amplification reaction) in the first reaction chamber 121 and the second reaction chamber 122 to achieve the nucleic acid detection of the sample. FIG. 5 is a bottom view illustrating a reaction portion according to some embodiments of the present disclosure. As shown in FIG. 2 and FIG. 5, the first reaction chamber 121 and the second reaction chamber 122 may be disposed side by side on the reaction portion 120, and the first reaction chamber 121 may not communicate with the second reaction chamber 122. For example, the first reaction chamber 121 and the second reaction chamber 122 may be groove structures disposed side by side on the reaction portion 120.

In some embodiments, a reaction reagent may be placed in the first reaction chamber 121 and the second reaction chamber 122 in advance. In some embodiments, a loop-mediated isothermal amplification reaction solution for housekeeping genes (e.g., a RnaseP gene loop-mediated isothermal amplification reaction solution) may be placed in the first reaction chamber 121 to make the sample to undergo the reverse transcription loop-mediated isothermal amplification reaction in the first reaction chamber 121 so as to detect an internal standard gene of the sample. In some embodiments, a formula of the reaction reagent may be selected as Bst DNA polymerase of 2-10 U, 2.5 µL of 10 x Bst Buffer, 0.5-3 µL of 10 x RnaseP Primers, 20-30 µL of template DNA, betaine of 0.1-10 mg/mL, Mg₂SO₄ of 2-10 mM, calcein of 0.01%-0.5%, etc. In some embodiments, a loop-mediated isothermal amplification reaction reagent (e.g., a novel coronavirus gene loop-mediated isothermal amplification reaction reagent) for viruses or bacteria to be detected may be placed in the second reaction chamber 122 to make the sample to undergo the reverse transcription loop-mediated isothermal amplification reaction in the second reaction chamber 122 so as to detect a target gene of the sample. In some embodiments, a formula of the reaction reagent may be selected as Bst DNA polymerase of 2-10 U, 2.5 µL of 10 x Bst Buffer, 0.5-3 µL of 10 x Covid-19 Primers, 20-30 µL of template DNA, betaine of 0.1-10 mg/mL, Mg₂SO₄ of 2-10 mM, calcein of 0.01 %-0.5%, etc. In some embodiments, when the detection of the internal standard gene of the sample performed in the first reaction chamber 121 includes different detection results (e.g., a negative result or a positive result), colors of first amplification reaction products generated by the sample may be different. In some embodiments, whether sampling succeeds or not and whether a detection reagent is in a normal response may be determined by observing the first amplification reaction product generated in the first reaction chamber 121 to determine the color of the first amplification reaction product generated in the first reaction chamber 121. For example, when the sample contains human-derived cells, the first amplification reaction product in the first reaction chamber 121 may be yellow (a positive result), indicating successful sampling and the normal response of the detection reagent. When the sample contains no human-derived cells, the first amplification reaction product in the first reaction chamber 121 may be pink (a negative result), indicating failed sampling or invalidation of the detection reagent. In some embodiments, when the first amplification reaction product within the first reaction chamber 121 is yellow (a positive result), colors of a second amplification reaction product generated within the second reaction chamber 122 may be different when the detection of the target gene of the sample performed in the second reaction chamber 122 includes different detection results (e.g., a negative result or a positive result). In some embodiments, results of the nucleic acid detection may be determined by observing the second amplification reaction product generated within the second reaction chamber 122 to determine the color of the second amplification reaction product generated within the second reaction chamber 122. For example, when the sample contains the virus or bacterium (e.g., novel coronavirus) to be detected, the color of the second amplification reaction product in the second reaction chamber 122 may be yellow, indicating that the detection result is positive. When the sample contains no virus or bacterium (e.g., novel coronavirus) to be detected, the color of the second amplification reaction product in the second reaction chamber 122 may be pink, indicating that the detection result is negative.

For purposes of illustration, when the color of the first amplification reaction product is yellow, the detection result of the internal standard gene is positive (i.e., the detection result of the target gene is credible). At this moment, the color of the second amplification reaction product may be further observed. If the color of the second amplification reaction product is pink, the detection result of the target gene may be negative. If the color of the second amplification reaction product is yellow, the detection result of the target gene may be positive. When the color of the first amplification reaction product is pink, the detection result of the internal standard gene is negative (i.e., the detection result of the target gene is not credible). At this moment, the color of the second amplification reaction product may not be used as the detection result. That is, the nucleic acid detection may be invalid. In some embodiments, the reaction portion 120 where the reaction reagent is dispensed in advance may be lyophilized in a lyophilizer.

By disposing the first reaction chamber 121 and the second reaction chamber 122 in the nucleic acid detection device 100, the reliability of the detection result of the target gene performed in the second reaction chamber 122 may be determined by the detection of the internal standard gene performed in the first reaction chamber 121, thereby improving the accuracy of the gene detection performed in the nucleic acid detection device 100, and thus avoiding a detection error caused by substandard sampling, sample contamination, etc.

Each of the first observation chamber 113 and the second observation chamber 114 may be a columnar structure with a hollow interior. The first observation chamber 113 may accommodate the fluid (e.g., the first amplification reaction product after the amplification reaction) after the reaction occurs in the first reaction chamber 121, and the second observation chamber 114 may accommodate the fluid (e.g., the second amplification reaction product after the amplification reaction) after the reaction occurs in the second reaction chamber 122. A bottom portion of each of the first observation chamber 113 and the second observation chamber 114 may comprise an inlet (e.g., a first inlet 113-1 and a second inlet 114-1 as described below). The first observation chamber 113 may communicate with the first reaction chamber 121 through an opening at the bottom portion of the first observation chamber 113, and the second observation chamber 114 may communicate with the second reaction chamber 122 through an opening at the bottom portion of the second observation chamber 114. In some embodiments, the first observation chamber 113 may comprise a first observation port 113-2, and the second observation chamber 114 may comprise a second observation port 114-2. In some embodiments, after the first amplification reaction product enters the first observation chamber 113, the first amplification reaction product within the first observation chamber 113 may be observed through the first observation port 113-2. For example, the color of the first amplification reaction product within the first observation chamber 113 may be observed through the first observation port 113-2. In some embodiments, after the second amplification reaction product enters the second observation chamber 113, the second amplification reaction product within the second observation chamber 114 may be observed through the second observation port 114-2. For example, the color of the second amplification reaction product within the second observation chamber 114 may be observed through the second observation port 114-2. In some other embodiments, the housing portion 110 may be transparent to allow an operator to observe the fluid within the first observation chamber 113 and the second observation chamber 114 through a housing. Results of the detection of the internal standard gene and the detection of the target gene may be determined by observing the color of the fluid (e.g., the first amplification reaction product) in the first observation chamber 113 and the color of the fluid (e.g., the second amplification reaction product) in the second observation chamber 114, thus simplifying the process of the detection result obtaining and improving the efficiency of the process of the detection result obtaining.

In some embodiments, the nucleic acid detection device may further comprise a piston assembly 130. The housing portion 110 may be further configured to include a piston chamber 112. The piston assembly 130 may be at least partially disposed in the piston chamber 112, and the piston chamber 112 may communicate with both the first reaction chamber 121 and the second reaction chamber 122. The piston assembly 130 may move in the piston chamber 112 so as to control the fluid containing the sample to enter the first reaction chamber 121 and the second reaction chamber 122 from the sample chamber 111, and control the fluid in the first reaction chamber 121 and the second reaction chamber 122 to respectively enter the first observation chamber 113 and the second observation chamber 114.

In some embodiments, when the piston assembly 130 (e.g., a piston) at least partially moves in a first direction in the piston chamber 112, the fluid containing the sample in the sample chamber 111 may enter the first reaction chamber 121 and the second reaction chamber 122. When the piston assembly 130 at least partially moves in a second direction in the piston chamber 112, the fluid in the first reaction chamber 121 may enter the first observation chamber 113, and the fluid in the second reaction chamber 122 may enter the second observation chamber 114.

In some embodiments, the first direction may be the same as the second direction. In some other embodiments, the first direction may be opposite to the second direction. That the first direction is the same as the second direction or the first direction is opposite to the second direction may be determined according to relative positions between the housing portion 110 and the reaction portion 120, and a flow direction of the fluid required between various chambers (e.g., the sample chamber 111, the first reaction chamber 121, the second reaction chamber 122, the first observation chamber 113, and the second observation chamber 114). That the reaction portion 120 is located at the bottom portion of the housing portion 110, and the first direction is opposite to the second direction may be taken as examples for illustration. For instance, as illustrated in FIG. 1, the first direction may be a direction away from the reaction portion 120 in a depth direction (i.e., a z-direction in FIG. 1) of the piston chamber 112, and the second direction may be a direction close to the reaction portion 120 in the depth direction (i.e., the z-direction in FIG. 1) of the piston chamber 112. In some embodiments, when the piston assembly 130 at least partially moves in the piston chamber 112 along the direction away from the reaction portion 120, the fluid containing the sample in the sample chamber 111 may enter the first reaction chamber 121 and the second reaction chamber 122 under a negative pressure, while the piston assembly 130 at least partially moves in the piston chamber 112 along the direction close to the reaction portion 120, the fluid (e.g. the amplified product after the amplification reaction) in the first reaction chamber 121 and the second reaction chamber 122 may enter the first observation chamber 113 and the second observation chamber 114, respectively, under the positive pressure.

The piston chamber 112 may be a columnar structure with a hollow interior. The piston chamber 112 may include at least part (e.g., a piston and a portion of a piston rod) of a structure of the piston assembly 130. A bottom portion of the piston chamber 112 may include two openings. The piston chamber 112 may communicate with the first reaction chamber 121 and the second reaction chamber 122 of the reaction portion 120, respectively, through the two openings at the bottom portion of the piston chamber 112.

In some other embodiments, the flow of the fluid containing the sample that enters the first reaction chamber 121 and the second reaction chamber 122 from the sample chamber 111, the flow of the fluid in the first reaction chamber 121 entering the first observation chamber 113, and the flow of the fluid in the second reaction chamber 122 entering the second observation chamber 114 may be controlled by a valve (e.g., a micro-electric valve).

In some embodiments, continuing to refer to FIGs. 1-3, the nucleic acid detection device 100 may further comprise an elastic membrane 160. The elastic membrane 160 may be disposed between the housing portion 110 and the reaction portion 120. In some embodiments, the elastic membrane 160 may be made of a double-sided adhesive material. The double-sided adhesive material may be subjected to partial viscosity removing operation, so that a one-way circulation function (referring to the relevant descriptions below for detail) may be achieved and a connection between the housing portion 110 and the reaction portion 120 may also be achieved.

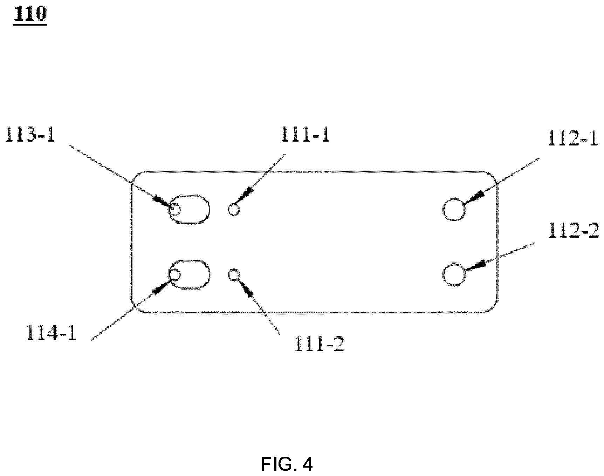

FIG. 4 is a bottom view illustrating a housing portion according to some embodiments of the present disclosure. In some embodiments, referring to FIGs. 1-4, the sample chamber 111 may be further described below. The sample chamber 111 may include a sample inlet 111-3, a first sample outlet 111-1, and a second sample outlet 111-2. The sample may be placed into the sample chamber 111 from the sample inlet 111-3 and discharged from the sample chamber 111 through the first sample outlet 111-1 and the second sample outlet 111-2. The sample chamber 111 may communicate with the first reaction chamber 121 through the first sample outlet 111-1, and the sample chamber 111 may communicate with the second reaction chamber 122 through the second sample outlet 111-2. A nucleic acid detection template obtained by lysis and release in the sample chamber 111 may enter the first reaction chamber 121 through the first sample outlet 111-1 and enter the second reaction chamber 122 through the second sample outlet 111-2 for further amplification reactions.

In some embodiments, the first reaction chamber 121 may communicate with the first sample outlet 111-1 by a first way valve structure 141. The first way valve structure 141 may allow the fluid containing the sample to enter the first reaction chamber 121 from the sample chamber 111. That is, the fluid containing the sample in the sample chamber 111 may flow into the first reaction chamber 121 through the first sample outlet 111-1, and the fluid containing the sample cannot flow back into the sample chamber 111 after entering the first reaction chamber 121. The second reaction chamber 122 may communicate with the second sample outlet 111-2 through a second way valve structure 142. The second way valve structure 142 may allow the fluid containing the sample to enter the second reaction chamber 122 from the sample chamber 111. That is, the fluid containing the sample in the sample chamber 111 may flow into the second reaction chamber 122 through the second sample outlet 111-2, and the fluid containing the sample cannot flow back into the sample chamber 111 after entering the second reaction chamber 122. By disposing the first way valve structure 141 and the second way valve structure 142, the fluid in the first reaction chamber 121 and the second reaction chamber 122 cannot flow into the sample chamber, avoiding different fluids from being mixed in the nucleic acid detection process and ensuring the accuracy of the nucleic acid detection. In some embodiments, the first way valve structure 141 and the second way valve structure 142 may be micro electronically controlled valves. In some other embodiments, the first way valve structure 141 and the second way valve structure 142 may be implemented by the elastic membrane 160, referring to the relevant descriptions below for detail.

In some embodiments, the sample inlet 111-3 may also be disposed with a sample chamber plug (not shown in the figure) for sealing the sample chamber 111. For instance, the sample chamber plug may block the sample inlet 111-3 to seal the sample chamber 111. Merely by way of example, when the nucleic acid detection device 100 is not in use, the sample chamber plug may seal the sample chamber 111 to prevent impurities (e.g., dust) in the external environment from damaging an environment in the sample chamber 111. As another example, the sample chamber plug may seal the sample chamber 111 after the sample is placed in the sample chamber 111, which may prevent overflow of the fluid within the sample chamber 111 and protect the environment within the sample chamber 111.

In some embodiments, referring to FIGs. 1-4, the first observation chamber 113 and the second observation chamber 114 are further described below. Each of the first observation chamber 113 and the second observation chamber 114 may have an inlet. The inlet of the first observation chamber 113 may be a first inlet 113-1, and the inlet of the second observation chamber 114 may be a second inlet 114-1. The first observation chamber 113 may communicate with the first reaction chamber 121 through the first inlet 113-1, and the second observation chamber 114 may communicate with the second reaction chamber 122 through the second inlet 114-1.

In some embodiments, the first inlet 113-1 may be disposed at the bottom portion of the first observation chamber 113, and the fluid (e.g., the first amplification reaction product) in the first reaction chamber 121 may enter the first observation chamber 113 through the first inlet 113-1. In some embodiments, the second inlet 114-1 may be disposed at the bottom portion of the second observation chamber 114, and the fluid (e.g., the second amplification reaction product) in the second reaction chamber 121 may enter the second observation chamber 114 through the second inlet 114-1. For instance, when the piston assembly 130 at least partially moves in the piston chamber 112 along the second direction (e.g. moving in the direction close to the reaction portion 120), the first amplification reaction product in the first reaction chamber 121 may enter the first observation chamber 113 through the first inlet 113-1 under a positive pressure, and the second amplification reaction product in the second reaction chamber 121 may enter the second observation chamber 113 through the second inlet 114-1 under the positive pressure.

In some embodiments, as illustrated in FIG. 3 and FIG. 5, the first reaction chamber 121 may include a first sub-chamber 1211 and a second sub-chamber 1212 connected sequentially in a length direction (i.e., an x-direction in FIG. 5). The first sub-chamber 1211 may communicate with the second sub-chamber 1212. In some embodiments, a width of the first sub-chamber 1211 may be less than a minimum width of the second sub-chamber 1212. The width of the first sub-chamber 1211 may refer to a dimension of the first sub-chamber 1211 in a width direction (i.e., a y-direction in FIG. 5). The width of the second sub-chamber 1212 may refer to a dimension of the second sub-chamber 1212 in the width direction (i.e., the y-direction in FIG. 5). In some embodiments, the width of the second sub-chamber 1212 may vary in the length direction, but the minimum width of the second sub-chamber 1212 may be still greater than the width of the first sub-chamber 1211. A depth of the first sub-chamber 1211 may be less than a depth of the second sub-chamber 1212. The depth of the first sub-chamber 1211 may refer to a dimension of the first sub-chamber 1211 in a depth direction (i.e., the z-direction in FIG. 3). The depth of the second sub-chamber 1212 may be a dimension of the second sub-chamber 1212 in the depth direction (i.e., the z-direction in FIG. 3). The first inlet 113-1 may arrange opposite to the first sub-chamber 1211. That is, the first inlet 113-1 may be opposite to a top opening of the first sub-chamber 1211. By disposing the dimensions of the first sub-chamber 1211 and the second sub-chamber 1212 in the manner described above and making the first inlet 113-1 arrange opposite to the first sub-chamber 1211, the first sub-chamber 1211 may have a relatively small width and a relatively small depth. Under a driving action of the piston assembly 130, the fluid (e.g., the first amplification reaction product) in the first sub-chamber 1211 may pass through the first inlet 113-1 to enter the first observation chamber 113 relatively easily.

In some embodiments, the width of the first sub-chamber 1211 may be substantially the same as a diameter of the first inlet 113-1. In some embodiments, the width of the first sub-chamber 1211 may be within a range from 1.0 mm to 1.5 mm, and the width of the second sub-chamber 1212 may be within a range from 1.5 mm to 2.0 mm. In some embodiments, the depth of the first sub-chamber 1211 may be within a range from 0.5mm to 1.0mm, and the depth of the second sub-chamber 1212 may be within a range from 1.5 mm to 2.0 mm.

In some embodiments, continuing to refer to FIG. 5, the second reaction chamber 122 may include a third sub-chamber 1221 and a fourth sub-chamber 1222 connected sequentially in the length direction (i.e., the x-direction in FIG. 5). The third sub-chamber 1221 may communicate with the fourth sub-chamber 1222. In some embodiments, a width of the third sub-chamber 1221 may be less than a minimum width of the fourth sub-chamber 1222. The width of the third sub-chamber 1221 may refer to a dimension of the third sub-chamber 1221 in the width direction (i.e., the y-direction in FIG. 5). The width of the fourth sub-chamber 1222 may refer to a dimension of the fourth sub-chamber 1222 in the width direction (i.e., the y-direction in FIG. 5). In some embodiments, the width of the fourth sub-chamber 1222 may vary in the length direction, but the minimum width of the fourth sub-chamber 1222 may be still greater than the width of the third sub-chamber 1221. A depth of the third sub-chamber 1221 may be less than a depth of the fourth sub-chamber 1222. The depth of the third sub-chamber 1221 may refer to a dimension of the third sub-chamber 1221 in the depth direction (i.e., the z-direction in FIG. 3, similar to the depth of the first sub-chamber 1211). The depth of the fourth sub-chamber 1222 may refer to a dimension of the fourth sub-chamber 1222 in the depth direction (i.e., the z-direction in FIG. 3, similar to the depth of the second sub-chamber 1212). The second inlet 114-1 may arrange opposite to the third sub-chamber 1221. That is, the second inlet 114-1 may be located opposite to a top opening of the third sub-chamber 1221. By disposing the dimensions of the third sub-chamber 1221 and the fourth sub-chamber 1222 in the manner described above and making the second inlet 114-1 arrange opposite to the third sub-chamber 1221, the third sub-chamber 1221 may have a relatively small width and a relatively small depth. Under the driving action of the piston assembly 130, the fluid (e.g., the second amplification reaction product) in the third sub-chamber 1221 may more easily pass through the second inlet 114-1 to enter the second observation chamber 114.

In some embodiments, the width of the third sub-chamber 1221 may be substantially the same as the diameter of the second inlet 114-1. In some embodiments, the width of the third sub-chamber 1221 may be within a range from 1.0 mm to 1.5 mm. The width of the fourth sub-chamber 1222 may be within a range from 1.5 mm to 2.0 mm. The depth of the third sub-chamber 1221 may be within a range from 0.5 mm to 1.0 mm. The width of the fourth sub-chamber 1222 may be within a range from 1.5 mm to 2.0 mm.

In some embodiments, the second sub-chamber 1212 may arrange opposite to the first sample outlet 111-1 of the sample chamber 111. That is, the first sample outlet 111-1 may be opposite to a top opening of the second sub-chamber 1212. In some embodiments, the fourth sub-chamber 1222 may arrange opposite to the second sample outlet 111-2 of the sample chamber 111. That is, the second sample outlet 111-2 may be opposite to a top opening of the fourth sub-chamber 1222.

In some embodiments, referring to FIGs. 1-4, a partition may be disposed in the piston chamber 112. The piston chamber 112 may be divided into a fifth sub-chamber 1121 and a sixth sub-chamber 1122 by the partition. In some embodiments, the partition within the piston chamber 112 may be disposed in the depth direction (e.g., the z-direction in FIG. 1 or FIG. 3) of the piston chamber 112 to divide the piston chamber 112 into the fifth sub-chamber 1121 and the sixth sub-chamber 1122 distributed side-by-side in the width direction (e.g., the y-direction in FIG. 1). In some embodiments, the fifth sub-chamber 1121 (e.g., a bottom portion of the fifth sub-chamber 1121) may include a first opening 112-1. The fifth sub-chamber 1121 may communicate with the first reaction chamber 121 of the reaction portion 120 through the first opening 112-1. The sixth sub-chamber 1122 (e.g., a bottom portion of the sixth sub-chamber 1122) may include a second opening 112-2. The sixth sub-chamber 1122 may communicate with the second reaction chamber 122 of the reaction portion 120 through the second opening 112-2.

In some embodiments, as illustrated in FIG. 5, the second sub-chamber 1212 may at least include a first portion 1212-1 and a second portion 1212-2. A width of the first portion 1212-1 may be larger than a width of the second portion 1212-2. The first opening 112-1 may arrange opposite to the second portion 1212-2. In some embodiments, the width of the second portion 1212-2 may be substantially the same as a diameter of the first opening 112-1. In some embodiments, the fourth sub-chamber 1222 may at least include a third portion 1222-1 and a fourth portion 1222-2. A width of the third portion 1222-1 may be larger than a width of the fourth portion 1222-2. The second opening may arrange opposite to the fourth portion 1222-2. In some embodiments, the width of the fourth portion 1222-2 may be substantially the same as the diameter of the second opening. It should be understood that, the widths of the first portion 1212-1, the second portion 1212-2, the third portion 1222-1, and the fourth portion 1222-2 may be understood as the dimensions of the above four portions in the width direction (i.e., the y-direction shown in FIG. 5). Through such an arrangement, the piston assembly 130 may be able to apply the positive or negative pressure to the first reaction chamber 121 and the second reaction chamber 122 more stably, and the first reaction chamber 121 and the second reaction chamber 122 may be able to maintain gas tightness better.

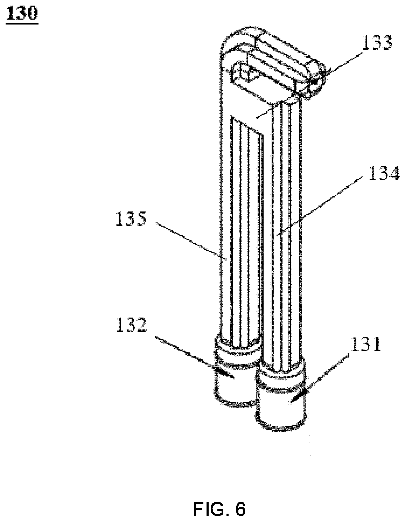

FIG. 6 is a schematic diagram illustrating an exemplary structure of a piston assembly according to some embodiments of the present disclosure. Referring to FIG. 2 and FIG. 6, the piston assembly 130 may comprise a first piston 131 and a second piston 132. The first piston 131 may be disposed within the fifth sub-chamber 1121 of the piston chamber 112, and the second piston 132 may be disposed within the sixth sub-chamber 1122 of the piston chamber 112. In some embodiments, the first piston 131 may move within the fifth sub-chamber 1121 of the piston chamber 112. When the first piston 131 moves within the fifth sub-chamber 1121, the first piston 131 may form a positive or negative pressure within the first reaction chamber 121, thereby driving the fluid to circulate between various chambers (e.g., the sample chamber 111 and the first reaction chamber 121, the first reaction chamber 121 and the first observation chamber 113). In some embodiments, the second piston 132 may move within the sixth sub-chamber 1122 of the piston chamber 112. When the second piston 132 moves within the sixth sub-chamber 1122, the second piston 132 may form a positive or negative pressure within the second reaction chamber 122, and the positive or negative pressure may act on the elastic membrane 160 to drive the fluid to circulate between various chambers (e.g., the sample chamber 111 and the second reaction chamber 122, the second reaction chamber 122 and the second observation chamber 114), referring to the relevant description below for detail.

In some embodiments, the movement of the first piston 131 in the fifth sub-chamber 1121 and the movement of the second piston 132 in the sixth sub-chamber 1122 may be controlled, respectively. In some embodiments, the first piston 131 and the second piston 132 may move synchronously. That is, the movement of the first piston 131 in the fifth sub-chamber 1121 and the movement of the second piston 132 in the sixth sub-chamber 1122 may be controlled in unison. For example, the piston assembly may further include a first piston rod 134, a second piston rod 135, and a connector 133. One end of the first piston rod 134 may connect to the first piston 131, one end of the second piston rod 135 may connect to the second piston 132, and the connector 133 may connect to both the other end of the first piston rod 134 and the other end of the second piston rod 135. Through such an arrangement, the first piston 131 and the second piston 132 may be controlled in unison, and the fluid containing the sample in the sample chamber 111 may be fed into the first reaction chamber 121 and the second reaction chamber 122 when the operator only operates one time (e.g., pulling the connector 133 in the first direction). After the fluid containing the sample undergoes the reactions within the first reaction chamber 121 and the second reaction chamber 122, when the operator only operates one time (e.g. pushing the connector133 in the second direction), the first amplification reaction product within the first reaction chamber 121 may be fed into the first observation chamber 113, and the second amplification reaction product within the second reaction chamber 122 may be fed into the second observation chamber 114.

In some embodiments, referring to FIG. 2 and FIG. 3, the first way valve structure 141 and the second way valve structure 142 may be formed by the elastic membrane 60. The elastic membrane 160 may be configured to include a first through hole 161 and a second through hole 162. The first through hole 161 may communicate with the first reaction chamber 121. For example, when the elastic membrane 160 is disposed on the reaction portion 120, the first through hole 161 may be located at a top opening of the first reaction chamber 121. The first through hole 161 may be staggered with the first sample outlet 111-1 of the sample chamber 111, so that the elastic membrane 160 and the first through hole 161 thereon may form the first way valve structure 141. The first through hole 161 being staggered with the first sample outlet 111-1 of the sample chamber 111 may be understood as that the first through hole 161 does not overlap with the first sample outlet 111-1, thereby making the first sample outlet 111-1 to be blocked by the elastic membrane 160. The relevant principles of the first way valve structure 141 may refer to the relevant description below. The second through hole 162 may communicate with the second reaction chamber 122. For example, when the elastic membrane 160 is disposed on the reaction portion 120, the second through hole 162 may be located at a top opening of the second reaction chamber 122. The second through hole 162 may be staggered with the second sample outlet 111-2 of the sample chamber 111, so that the elastic membrane 160 and the second through hole 162 thereon may form the second way valve structure 142. The second through hole 162 being staggered with the second sample outlet 111-2 of the sample chamber 111 may be understood as that the second through hole 162 does not overlap with the second sample outlet 111-2, thereby making the second sample outlet 111-2 to be blocked by the elastic membrane 160. The principles of the second way valve structure 142 may be similar to those of the first way valve structure 141.

In some embodiments, when the elastic membrane 160 is in an initial state, the elastic membrane 160 may block the first sample outlet 111-2 and the second sample outlet 111-2. When the piston assembly 130 at least partially moves in the piston chamber 112 along the first direction (e.g. the direction away from the reaction portion 120), a negative pressure may be formed in both the first reaction chamber 121 and the second reaction chamber 122, making the elastic membrane 160 to deform under the negative pressure (e.g. deform toward the first reaction chamber 121 and the second reaction chamber 122), thereby making the first sample outlet 111-1 and the second sample outlet 111-2 to change from a state in which the first sample outlet and the second sample outlet are blocked by the elastic membrane 160 to a state in which the fluid containing the sample is allowed to flow out. At this moment, the first way valve structure 141 may allow the fluid containing the sample to flow from the sample chamber 111 to the first reaction chamber 121, and the second way valve structure 142 may allow the fluid containing the sample to flow from the sample chamber 111 to the second reaction chamber 122.

In some embodiments, the second sub-chamber 1212 may communicate with the first sample outlet 111-1 of the sample chamber 111 via the first way valve structure 141. For example, when the elastic membrane 160 is disposed on the reaction portion 120, the first sample outlet 111-1 and the first through hole 161 may both be located at the top opening of the second sub-chamber 1212. In some embodiments, the fourth sub-chamber 1222 may communicate with the second sample outlet 111-2 of the sample chamber 111 by the second way valve structure 142. For example, when the elastic membrane 160 is disposed on the reaction portion 120, the second sample outlet 111-2 and the second through hole 162 may both be located at the top opening of the fourth sub-chamber 1222.

In some embodiments, the elastic membrane 160 may be configured to include a third through hole 163 and a fourth through hole 164. The first inlet 113-1 may communicate with the first reaction chamber 121 through the third through hole 163, and the second inlet 114-1 may communicate with the second reaction chamber 122 through the fourth through hole 164. For example, when the elastic membrane 160 is disposed on the reaction portion 120, the third through hole 163 may be located at the top opening of the first reaction chamber 121 and the fourth through hole 164 may be located at the top opening of the second reaction chamber 122. In some embodiments, the third through hole 163 may arrange opposite to the first inlet 113-1, so that the elastic membrane 160 and the third through hole 163 thereon may form a third way valve structure 143. The fourth through hole 164 may arrange opposite to the second inlet 114-1, so that the elastic membrane 160 and the fourth through hole 164 thereon may form a fourth way valve structure 144.

In some embodiments, when the piston assembly 130 at least partially moves in the piston chamber 112 along the second direction (e.g., the direction close to the reaction portion 120), a positive pressure may be formed both in the first reaction chamber 121 and the second reaction chamber 122. The fluid in the first reaction chamber 121 may enter the first observation chamber 113 under the positive pressure through the third way valve structure 143 and the first inlet 113-1, and the fluid in the second reaction chamber 122 may enter the second observation chamber 114 under the positive pressure through the fourth way valve structure 144 and the second inlet 114-1.

In some embodiments, referring to FIG. 3 and FIG. 5, the third through hole 163 may be disposed correspondence to the first sub-chamber 1211 of the first reaction chamber 121 to facilitate that the fluid (e.g., the first amplification reaction product) in the first reaction chamber 121 flows into the first observation chamber 113 through the first sub-chamber 1211 and the third through hole 163. The fourth through hole 164 may be disposed correspondence to the third sub-chamber 1221 of the second reaction chamber 122 to facilitate that the fluid (e.g., the second amplification reaction product) in the second reaction chamber 122 to flow into the second observation chamber 114 through the third sub-chamber 1213 and the fourth through hole 164.

In some embodiments, the elastic membrane 160 may also be configured to include a fifth through hole 165 and a sixth through hole 166. The piston chamber 112 (e.g., the first opening 112-1 of the piston chamber 112) may communicate with the first reaction chamber 121 through the fifth through hole 165, and the piston chamber 112 (e.g., the second opening 112-2 of the piston chamber 112) may communicate with the second reaction chamber 122 through the sixth through hole 166. In some embodiments, when the piston assembly 130 at least partially moves within the piston chamber 112 to apply the negative or positive pressure to the piston chamber 112, the negative or positive pressure may act on the first reaction chamber 121 through the first opening 112-1 and the fifth through hole 165, and act on the second reaction chamber 122 through the second opening 112-2 and the sixth through hole 166, thereby making the fluid to flow in the different chambers.

In some embodiments, continuing to refer to FIG. 2, the fifth through hole 165 of the elastic membrane 160 may be configured to include a hydrophobic breathable membrane 150. In some embodiments, the sixth through hole 166 of the elastic membrane 160 may be configured to include the hydrophobic breathable membrane 150. The hydrophobic permeable membrane 150 may allow air to pass through and prevent liquid from passing through. By disposing the hydrophobic breathable membrane 150, the positive or negative pressure generated by the movement of the piston assembly 130 in the piston chamber 112 may act on the reaction portion 120 through the hydrophobic breathable membrane 150, while also preventing the fluid in the first reaction chamber 121 or the second reaction chamber 122 from flowing into the piston chamber 112.

In some embodiments, the nucleic acid detection device 100 may also include a sealing membrane 170. The sealing membrane 170 may seal the nucleic acid detection device 100, so that the nucleic acid detection device 100 forms a fully enclosed structure. The use of the sealing membrane 170 in combination with components such as the sample chamber plug may also prevent cross-contamination between the chambers of the nucleic acid detection device 100 and cross-contamination between the chambers and the external environment.

In some embodiments, the nucleic acid detection device may also include a heating assembly (not shown in the figures) disposed at the bottom portion of the reaction portion 120. The heating assembly may heat the first reaction chamber 121 and the second reaction chamber 122, thereby providing a suitable reaction temperature for the amplification reaction performed in the first reaction chamber 121 and the second reaction chamber 122 to achieve the amplification reaction of the nucleic acid detection template. The heating assembly may include a resistance heating assembly, an infrared heating assembly, etc. In some embodiments, the nucleic acid detection device 100 may also be disposed with no heating assembly, but the reaction portion 120 may be heated by an external heating device (e.g., a thermostatic metal bath device) to provide the suitable reaction temperature.

In some embodiments, a dimension of the nucleic acid detection device 100 in the length direction (i.e., the x-direction in FIG. 1) may be within a range from 35 to 48 mm. In some embodiments, a dimension of the nucleic acid detection device 100 in the width direction (i.e., the y-direction in FIG. 1) may be within a range from 10 to 14 mm. In some embodiments, a dimension of the nucleic acid detection device 100 in the depth direction (i.e., the z-direction in FIG. 1) may be within a range from 50 to 55 mm. A size of the nucleic acid detection device may be small, which is convenient to carry and transport.

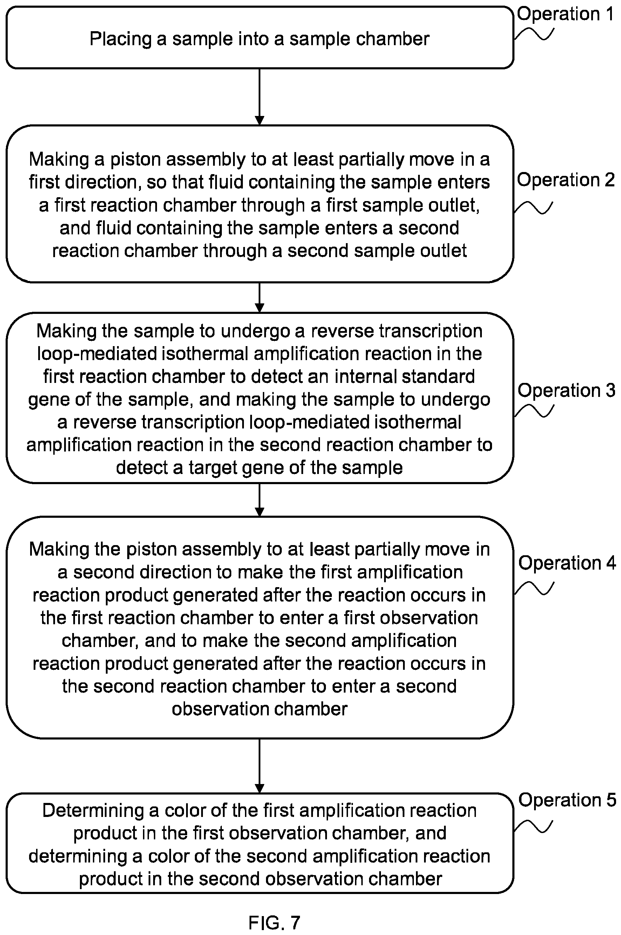

In some embodiments, the present disclosure further provides a nucleic acid detection method. The nucleic acid detection method may use the nucleic acid detection device 100 described in any of the above technical solutions to achieve the nucleic acid detection on viruses or bacteria. FIG. 7 is a flowchart illustrating a nucleic acid detection process according to some embodiments of the present disclosure. The nucleic acid detection process may include one or more of the following operations.

In operation 1, a sample may be placed into the sample chamber 111.

In some embodiments, the sample may be a nasal swab sample, a pharyngeal swab sample, etc. In some embodiments, a sample release agent may be pre-stored within the sample chamber 111. In some embodiments, a nasal swab sample or pharyngeal swab sample may be collected and placed in the sample chamber 111 and the nasal swab sample or the pharyngeal swab sample may be immersed with the sample release agent for repeated swabbing (e.g., about 15 times), and then the swab may be removed. The sample chamber may be covered with a sample chamber plug. The sample may be uniformly mixed with the sample release agent. In some embodiments, after the sample is mixed with the sample release agent, standing for 1-20 minutes may be performed to allow the sample to undergo sufficient lysis and release to obtain a nucleic acid detection template. In some embodiments, after the sample is mixed with the sample release agent, standing for 5-10 minutes may be performed.

In operation 2, the piston assembly 130 may be made to at least partially move in a first direction, so that fluid containing the sample enters the first reaction chamber 121 through the first sample outlet 111-1, and fluid containing the sample enters the second reaction chamber 122 through the second sample outlet 111-2.

In some embodiments, the first direction may refer to a direction away from the reaction portion 120 along the piston chamber 112. The piston assembly 130 (e.g., the first piston 131 and the second piston 132) may be pulled in the direction away from the reaction portion 120, making the fluid containing the sample obtained in operation 2 (i.e., the fluid containing the nucleic acid detection template) to enter the first reaction chamber 121 and the second reaction chamber 122, respectively.

In operation 3, the sample may be made to undergo a reverse transcription loop-mediated isothermal amplification reaction in the first reaction chamber 121 to detect an internal standard gene of the sample, and the sample may be made to undergo a reverse transcription loop-mediated isothermal amplification reaction in the second reaction chamber 122 to detect a target gene of the sample.

It should be understood that, the reverse transcription refers to a process of synthesizing DNA using RNA as a template. The loop-mediated isothermal amplification reaction is a novel isothermal nucleic acid amplification manner. The feature of the loop-mediated isothermal amplification reaction may include that four specific primers are designed for six regions of a target gene, constant temperature amplification is performed under the action of strand displacement DNA polymerase (Bst DNA polymerase), and 10⁹ to 10¹⁰ times of nucleic acid amplification is achieved in a short time (e.g., about 15-60 minutes). In some embodiments, the first reaction chamber 121 may be pre-loaded with a reverse transcription loop-mediated isothermal amplification lyophilization reaction reagent for housekeeping genes, such as, the RnaseP gene loop-mediated isothermal amplification lyophilization reaction reagent formula described above. In some embodiments, in operation 3, the nucleic acid detection device 100 may be placed on a heating device (e.g., a thermostatic metal bath device) for heating to initiate the nucleic acid isothermal amplification reaction, making the reverse transcription loop-mediated isothermal amplification reaction to occur within the first reaction chamber 121 to obtain a first amplification reaction product. In some embodiments, the nucleic acid detection device 100 may be heated at a temperature range from 50°C to 75°C. In some embodiments, the nucleic acid detection device 100 may be heated at a temperature range from 60°C to 65°C. In some embodiments, the nucleic acid detection device 100 may be heated for 5 to 50 minutes. That is, the amplification reaction time may be within a range of 5 to 50 minutes. In some embodiments, the nucleic acid detection device 100 may be heated for 30 to 40 minutes.

In some embodiments, the second reaction chamber 122 may be pre-loaded with a reverse transcription loop-mediated isothermal amplification lyophilization reaction reagent for virus or bacterium to be detected, such as, the reverse transcription loop-mediated isothermal amplification lyophilization reaction reagent formula for the novel coronavirus described above. In some embodiments, in operation 3, since the nucleic acid detection device 100 is placed on the heating device (e.g., the thermostatic metal bath) for heating to initiate the nucleic acid isothermal amplification reaction, the reverse transcription loop-mediated isothermal amplification reaction may occur within the second reaction chamber 122 to obtain a second amplification reaction product.

In operation 4, the piston assembly 130 may be made to at least partially move in a second direction to make the first amplification reaction product generated after the reaction occurs in the first reaction chamber 121 to enter a first observation chamber 113, and to make the second amplification reaction product generated after the reaction occurs in the second reaction chamber 122 to enter a second observation chamber 114.

In some embodiments, the second direction may refer to a direction close to the reaction portion 120 along the piston chamber 112. In some embodiments, after the nucleic acid detection device 100 is removed from the heating device, the piston assembly 130 (e.g., the first piston 131 and the second piston 132) may be pushed toward the reaction portion 120, so that the first amplification reaction product obtained in operation 3 enters the first observation chamber 113, and the second amplification reaction product obtained in operation 3 enters the second observation chamber 114.

In operation 5, a color of the fluid in the first observation chamber 113 may be determined, and a color of the fluid in the second observation chamber 114 may be determined.

In some embodiments, the color of the first amplification reaction product in the first observation chamber 113 may be observed with naked eyes through the first observation port 113-2 to determine a detection result of the internal standard gene, and the color of the second amplification reaction product in the second observation chamber 114 may be observed with the naked eyes through the second observation port 114-2 to determine a detection result of the target gene. Thus, a nucleic acid detection result may be determined based on the detection of the internal standard gene and the detection of the target gene. More descriptions regarding the determination of the detection result may be referred to the relevant description above.

In some embodiments, the nucleic acid detection device 100 and the nucleic acid detection method provided by some embodiments of the present disclosure may be used to detect viruses, bacteria, etc., such as, viruses or bacteria from human bodies or animal bodies. In some embodiments, the nucleic acid detection device 100 and the nucleic acid detection method provided by some embodiments of the present disclosure may be used to detect novel coronaviruses.

The following are the process, result determination criteria, detection results, and analytical conclusions of the nucleic acid detection performed based on five negative samples (N1-N5) and five positive samples (P1-P5) by utilizing the nucleic acid detection device and the nucleic acid detection method described above. The five negative samples were all nasal swab samples or pharyngeal swab samples collected from healthy individuals using nasopharyngeal swabs or oropharyngeal swabs. The five positive samples were all pseudovirus nucleic acid standard substances of novel coronavirus (SARS-CoV-2).

The process of the nucleic acid detection was as follows. Firstly, the sample chamber plug of the sample chamber 111 was opened, 200 uL of the pseudovirus nucleic acid standard substances of novel coronavirus (SARS-CoV-2) were taken and placed into the sample chamber 111, the sample was uniformly mixed with the sample release agent by repeated blowing and beating with a pipette, and the sample chamber was covered with the sample chamber plug to stand for 5-10 minutes at the room temperature to obtain the sample release agent containing the nucleic acid template. Then, the piston assembly 130 was pulled in the first direction so that the sample release agent containing the nucleic acid template entered the first reaction chamber 121 and the second reaction chamber 122, respectively, and the entire nucleic acid detection device 100 was placed on the thermostatic metal bath device for heating (the heating temperature of 60°C-65°C) to initiate the nucleic acid isothermal amplification reaction, and the amplification reaction lasting for 30-40 minutes. Thereafter, when the amplification reaction is finished, the nucleic acid detection device was removed and the piston assembly 130 was pushed in the second direction. The first amplification reaction product in the first reaction chamber 121 entered the first observation chamber 113, and the second amplification reaction product in the second reaction chamber 122 entered the second observation chamber 114. The colors of the first amplification reaction product and the second amplification reaction product were observed with the naked eyes to determine the nucleic acid detection result.

The result determination criteria were as shown in the following table:

| Serial number | Second observation chamber | First observation chamber | Detection result |
|---|---|---|---|
| 1 | Pink | Yellow | Negative |
| 2 | Yellow | Yellow | Positive |
| 3 | Pink | Pink | Invalid detection |
| 4 | Yellow | Pink | Invalid detection |

The detection results were as shown in the following table:

| Serial number | Second observation chamber | First observation chamber | Detection result |
|---|---|---|---|
| N1 | Pink | Yellow | Negative |
| N2 | Pink | Yellow | Negative |
| N3 | Pink | Yellow | Negative |
| N4 | Pink | Yellow | Negative |
| N5 | Pink | Yellow | Negative |
| P1 | Yellow | Yellow | Positive |
| P2 | Yellow | Yellow | Positive |
| P3 | Yellow | Yellow | Positive |
| P4 | Yellow | Yellow | Positive |
| P5 | Yellow | Yellow | Positive |

The analytical conclusion was that: through the above experiments, it can be learned that the nucleic acid detection of the novel coronavirus was performed by applying the nucleic acid detection device and the nucleic acid detection method above, and the detection was accurate. In addition, it can be seen that through the operation process, the operation of the nucleic acid detection is convenient and the detection efficiency is high, and the detection result is generally available within 1 hour.

Having thus described the basic concepts, it may be rather apparent to those skilled in the art after reading this detailed disclosure that the foregoing detailed disclosure is intended to be presented by way of example only and is not limiting. Various alterations, improvements, and modifications may occur and are intended for those skilled in the art, though not expressly stated herein. These alterations, improvements, and modifications are intended to be suggested by this disclosure, and are within the spirit and scope of the exemplary embodiments of this disclosure.

Moreover, certain terminology has been used to describe embodiments of the present disclosure. For example, the terms "one embodiment," "an embodiment," and/or "some embodiments" mean that a particular feature, structure, or characteristic described in connection with the embodiment is included in at least one embodiment of the present disclosure. Therefore, it is emphasized and should be appreciated that two or more references to "an embodiment" or "one embodiment" or "an alternative embodiment" in various portions of this disclosure are not necessarily all referring to the same embodiment. Furthermore, the particular features, structures, or characteristics may be combined as suitable in one or more embodiments of the present disclosure.

Furthermore, the recited order of processing elements or sequences, or the use of numbers, letters, or other designations therefore, is not intended to limit the claimed processes and methods to any order except as may be specified in the claims. Although the above disclosure discusses through various examples what is currently considered to be a variety of useful embodiments of the disclosure, it is to be understood that such detail is solely for that purpose, and that the appended claims are not limited to the disclosed embodiments, but, on the contrary, are intended to cover modifications and equivalent arrangements that are within the spirit and scope of the disclosed embodiments. For example, although the implementation of various components described above may be embodied in a hardware device, it may also be implemented as a software only solution, e.g., an installation on an existing server or mobile device.

Similarly, it should be appreciated that in the foregoing description of embodiments of the present disclosure, various features are sometimes grouped together in a single embodiment, figure, or description thereof for the purpose of streamlining the disclosure aiding in the understanding of one or more of the various inventive embodiments. This method of disclosure, however, is not to be interpreted as reflecting an intention that the claimed subject matter requires more features than are expressly recited in each claim. Rather, inventive embodiments lie in less than all features of a single foregoing disclosed embodiment.

In some embodiments, the numbers expressing quantities or properties used to describe and claim certain embodiments of the application are to be understood as being modified in some instances by the term "about," "approximate," or "substantially." For example, "about," "approximate," or "substantially" may indicate ±20% variation of the value it describes, unless otherwise stated. Accordingly, in some embodiments, the numerical parameters set forth in the written description and attached claims are approximations that may vary depending upon the desired properties sought to be obtained by a particular embodiment. In some embodiments, the numerical parameters should be construed in light of the number of reported significant digits and by applying ordinary rounding techniques. Notwithstanding that the numerical ranges and parameters setting forth the broad scope of some embodiments of the application are approximations, the numerical values set forth in the specific examples are reported as precisely as practicable.

Each of the patents, patent applications, publications of patent applications, and other material, such as articles, books, specifications, publications, documents, things, and/or the like, referenced herein is hereby incorporated herein by this reference in its entirety for all purposes, excepting any prosecution file history associated with same, any of same that is inconsistent with or in conflict with the present document, or any of same that may have a limiting effect as to the broadest scope of the claims now or later associated with the present document. By way of example, should there be any inconsistency or conflict between the description, definition, and/or the use of a term associated with any of the incorporated material and that associated with the present document, the description, definition, and/or the use of the term in the present document shall prevail.

In closing, it is to be understood that the embodiments of the application disclosed herein are illustrative of the principles of the embodiments of the application. Other modifications that may be employed may be within the scope of the application. Thus, by way of example, but not of limitation, alternative configurations of the embodiments of the application may be utilized in accordance with the teachings herein. Accordingly, embodiments of the present application are not limited to that precisely as shown and described.

## Claims

1. A nucleic acid detection device, comprising a housing portion and a reaction portion, the housing portion being configured to include a sample chamber, a first observation chamber, and a second observation chamber, and the reaction portion being configured to include a first reaction chamber and a second reaction chamber; the sample chamber communicating with the first reaction chamber, the sample chamber communicating with the second reaction chamber; the first observation chamber communicating with the first reaction chamber, and the second observation chamber communicating with the second reaction chamber.

2. The nucleic acid detection device of claim 1, wherein the nucleic acid detection device further comprises a piston assembly, the housing portion is further configured to include a piston chamber, the piston assembly is at least partially disposed in the piston chamber, and the piston chamber communicates with both the first reaction chamber and the second reaction chamber; wherein:
the piston assembly moves in the piston chamber so as to control fluid containing a sample to enter the first reaction chamber and the second reaction chamber from the sample chamber, and control the fluid in the first reaction chamber and the second reaction chamber to respectively enter the first observation chamber and the second observation chamber.

3. The nucleic acid detection device of claim 2, wherein the nucleic acid detection device further comprises an elastic membrane, the elastic membrane being disposed between the housing portion and the reaction portion.

4. The nucleic acid detection device of claim 3, wherein the sample chamber includes a sample inlet, a first sample outlet, and a second sample outlet; wherein
the first reaction chamber communicates with the first sample outlet through a first way valve structure, the first way valve structure allowing the fluid containing the sample to enter the first reaction chamber from the sample chamber;
the second reaction chamber communicates with the second sample outlet through a second way valve structure, the second way valve structure allowing the fluid containing the sample to enter the second reaction chamber from the sample chamber.

5. The nucleic acid detection device of claim 4, wherein the elastic membrane is configured to include a first through hole and a second through hole; wherein
the first through hole communicates with the first reaction chamber, the first through hole is staggered with the first sample outlet, and the elastic membrane and the first through hole thereon form the first way valve structure; and
the second through hole communicates with the second reaction chamber, the second through hole is staggered with the second sample outlet, and the elastic membrane and the second through hole thereon form the second way valve structure.

6. The nucleic acid detection device of claim 3, wherein an inlet of the first observation chamber communicates with the first reaction chamber, and an inlet of the second observation chamber communicates with the second reaction chamber;
the elastic membrane is configured to include a third through hole and a fourth through hole;
the inlet of the first observation chamber communicates with the first reaction chamber through the third through hole; and the inlet of the second observation chamber communicates with the second reaction chamber through the fourth through hole.

7. The nucleic acid detection device of claim 1, wherein the first reaction chamber comprises a first sub-chamber and a second sub-chamber connected sequentially in a length direction, a width of the first sub-chamber being less than a minimum width of the second sub-chamber, a depth of the first sub-chamber being less than a depth of the second sub-chamber, and an inlet of the first observation chamber arranging opposite to the first sub-chamber; and/or
the second reaction chamber comprises a third sub-chamber and a fourth sub-chamber connected sequentially in the length direction, a width of the third sub-chamber being less than a minimum width of the fourth sub-chamber, a depth of the third sub-chamber being less than a depth of the fourth sub-chamber, and an inlet of the second observation chamber arranging opposite to the third sub-chamber.

8. The nucleic acid detection device of claim 2, wherein a partition is disposed in the piston chamber, the piston chamber is divided into a fifth sub-chamber and a sixth sub-chamber by the partition, and the piston assembly comprises a first piston and a second piston, the first piston being disposed in the fifth sub-chamber, and the second piston being disposed in the sixth sub-chamber; wherein
the fifth sub-chamber has a first opening, the first opening communicating with the first reaction chamber; and the sixth sub-chamber has a second opening, the second opening communicating with the second reaction chamber.

9. The nucleic acid detection device of claim 8, wherein the piston assembly further comprises a first piston rod, a second piston rod, and a connector, one end of the first piston rod connecting to the first piston, one end of the second piston rod connecting to the second piston, and the connector connecting to both the other end of the first piston rod and the other end of the second piston rod.

10. The nucleic acid detection device of claim 3, wherein the elastic membrane is configured to include a fifth through hole and a sixth through hole, the first reaction chamber communicating with the piston chamber through the fifth through hole, and the second reaction chamber communicating with the piston chamber through the sixth through hole.

11. The nucleic acid detection device of claim 10, wherein a hydrophobic breathable membrane is disposed at the fifth through hole; and/or a hydrophobic breathable membrane is disposed at the sixth through hole.

12. A nucleic acid detection method, utilizing the nucleic acid detection device according to any one of claims 1-11, the detection method comprising:
placing a sample into the sample chamber;
making fluid containing the sample to enter the first reaction chamber through the first sample outlet, and making the fluid containing the sample to enter the second reaction chamber through the second sample outlet;
making the sample to undergo a reverse transcription loop-mediated isothermal amplification reaction within the first reaction chamber to detect an internal standard gene of the sample, and making the sample to undergo a reverse transcription loop-mediated isothermal amplification reaction within the second reaction chamber to detect a target gene of the sample;
making a first amplification reaction product generated after the reverse transcription loop-mediated isothermal amplification reaction occurs in the first reaction chamber to enter the first observation chamber, and making the second amplification reaction product generated after the reverse transcription loop-mediated isothermal amplification reaction occurs in the second reaction chamber to enter the second observation chamber; and
determining a color of the first amplification reaction product in the first observation chamber, and determining a color of the second amplification reaction product in the second observation chamber.
